# EUROPEAN PATENT APPLICATION

(11) **EP 2 757 377 A1**
(43) Date of publication of application: **23.07.2014**
(21) Application number: 12832566.9
(22) Date of filing: 06.09.2012
(51) Int. Cl.: G01N 33/574, G01N 33/543

(54) **METHOD FOR DETECTING PANCREATIC DISEASE MARKER**

(30) Priority: 13.09.2011 JP 2011199684
(71) Applicant: Olympus Corporation, Shibuya-ku Tokyo 151-0072 (JP)
(72) Inventor: SANUKI, Hiromi, Tokyo 151-0072 (JP); MORIYA, Nao, Tokyo 151-0072 (JP); KATAOKA, Rie, Tokyo 151-0072 (JP)
(74) Representative: Schicker, Silvia
(86) International application number: PCT/JP2012/072712
(87) International publication number: WO 2013/038981

(57) **Abstract**

The present invention relates to a method for detecting a marker for pancreatic disease for obtaining highly reliable results while reducing the risk of complications. It also relates to a method for a detecting a marker for pancreatic disease comprising: measuring the concentration of a marker for pancreatic disease in duodenal juice that is collected from a subject and contains spontaneously discharged pancreatic juice, by a measurement method including detecting antigen or antibody immobilized on a solid phase using an antigen-antibody reaction, and the aforementioned method for detecting a marker for pancreatic disease further including judging the subject to have the possibility of being afflicted with a pancreatic disease in the case the concentration of the marker for pancreatic disease is equal to or greater than a prescribed threshold value.

## Description

### TECHNICAL FIELD

The present invention relates to a method for detecting a marker for pancreatic disease discharged from the pancreas both safely and with a high level of performance.

The present application claims priority on the basis of Japanese Patent Application No. 2011-199684, filed in Japan on September 13, 2011, the contents of which are incorporated herein by reference.

### BACKGROUND ART

Pancreatic cancer has the poorest prognosis among cancers, having a five-year survival rate of less than 5%. One reason for this is that there is no effective examination method for diagnosing pancreatic cancer. Secondly, pancreatic cancer presents highly imperceptible symptoms in its early stages, and is frequently only discovered after having progressed to an advanced stage. Consequently, the prognosis of pancreatic cancer is expected to be greatly improved if it were possible to detect early, treatable pancreatic cancer by a lowly invasive and simple method that can be performed during routine medical examinations.

Bodily fluids serve as important biological samples for determining the states of various organs. Pancreatic juice is also an important biological sample for determining the state of the pancreas, and is used in cytodiagnosis, bicarbonate assay, bacteriological examinations and the like. In addition, wide-ranging searches for novel markers for use in pancreatic cancer and other pancreas-related diseases are being made in research fields.

Carcinoembryonic antigen (CEA), which is known to be a serum tumor marker, is a glycoprotein present on the surface of cancer cells that is also known to be highly expressed in pancreatic cancer cells. Determination of degree of malignancy and confirmation of therapeutic efficacy have been reported to be possible by measuring CEA concentration in serum during clinical laboratory testing. However, it is not possible to specify the organ where the cancer has originated simply by measuring CEA concentration in serum. In addition, although perhaps due to the concentration of tumor marker molecules being diluted by systemic blood, detection sensitivity in the case of pancreatic cancer is low at roughly 50%.

On the other hand, research has been reported relating to measuring CEA present in pancreatic juice that is discharged from the pancreas and contains pancreatic cancer cells. For example, research is being conducted by Rolny, et al. that attempts to differentiate between healthy subj ects and patients with chronic pancreatitis or pancreatic cancer using the method explained below (see Non-Patent Document 1). In this method, pancreatic juice and bile secretion stimulators (secretin and CCK) are first administered to test subjects by intravenous injection. Next, pancreatic juice discharged after 30 minutes and 45 minutes have elapsed following intravenous injection is collected from a tube fluoroscopically inserted into the duodenum (secretin test). Finally, CEA in the pancreatic juice obtained in this manner is measured. According to this method, CEA concentrations have been reported to be 3 ng/mL to 25 ng/mL in healthy subjects, and in the vicinity of 50 ng/mL in pancreatic cancer patients. However, it is difficult to judge these values to be accurate due to the extremely small numbers of cases of pancreatic cancer described in this report. In addition, since there also cases in which CEA concentrations are high in patients afflicted with diseases other than pancreatic cancer, a high CEA concentration in duodenal juice collected following administration of a secretion stimulator cannot necessarily be said to indicate the presence of pancreatic cancer. In addition, CEA in pancreatic juice similarly obtained by the secretin method has also been measured by Farini, et al., and concentrations in healthy subjects were reported to be 59 ng/mL to 450 ng/mL, while those in pancreatic cancer patients were reported to be 320 ng/mL to 800 ng/mL (see Non-Patent Document 2). In other words, despite having performed the same secretin test, there is nearly a ten-fold difference in CEA concentrations between the results reported by Rolny et al. and the results reported by Farini, et al. , thus indicating the problem of testing performance on pancreatic juice obtained with this secretin test varying considerably depending on the manner in which it is performed.

In response thereto, Non-Patent Documents 3 and 4 report results of inserting a catheter into the pancreatic duct during endoscopic retrograde cholangiopancreatography (ERCP), followed by collecting pancreatic juice discharged from the pancreas after administering a pancreatic juice secretion stimulator (secretin) for 10 minutes or 15 minutes, and measuring the concentration of CEA in the resulting pancreatic juice. Measured CEA concentrations in Non-Patent Document 3 were reported to be 0.1 ng/mL to 10 ng/mL in healthy subjects (control) and 1 ng/mL to 500 ng/mL in pancreatic cancer patients, while those in Non-Patent Document 4 were reported to be 56±26 ng/mL in healthy subjects (control) and 138±51 ng/mL in pancreatic cancer patients, with both reports indicating roughly similar values. In addition, high values were reported for pancreatic cancer testing performance, consisting of sensitivity of 71.4% and specificity of 93.3%. In other words, similar levels of CEA testing performance were demonstrated to able to be obtained even between different institutions by inserting a catheter into the pancreatic duct and using the pure pancreatic juice collected therefrom as a measurement sample. This is thought to be the result of measured values not being affected by bile and the like as well as being less susceptible to variations in collection technique in comparison with the previously described secretin test since pancreatic juice is collected by inserting a catheter directly into the pancreatic duct. However, this method requires the catheter to be inserted into the pancreatic duct endoscopically, thereby requiring a high skill level of the physician. In addition, since there is also the risk of serious symptoms associated with acute pancreatitis occurring as complications of the test, it can only be performed on patients for which pancreatic disease is strongly suspected.

### Prior Art Documents

### Non-Patent Documents

Non-Patent Document 1: Rolny, et al., Scandinavian Journal of Gastroenterology, 1977, Vol. 12, pp. 759-763
Non-patent Document 2: Farini, et al., Hepato-Gastroenterology, 1980, Vol. 27, pp. 213-216
Non-Patent Document 3: Pancreas, 1994, Vol. 9, No. 6, pp. 741-747
Non-Patent Document 4: Hepato-Gastroenterology, 1999, Vol. 46, No. 46, pp. 31-37

### DISCLOSURE OF THE INVENTION

### Problems to be Solved by the Invention

An object of an aspect of the present invention is to provide a method for detecting a marker for pancreatic disease for obtaining highly reliable results while reducing the risk of complications.

### Means for Solving the Problems

As a result of conducting extensive studies to solve the aforementioned problems, the inventors of the present invention found that, in the case of measuring CEA concentration in duodenal juice, namely in the case of measuring CEA concentration in pancreatic juice discharged into the duodenum, without administering a pancreatic juice secretion stimulator, CEA testing performance can still be obtained that is comparable to that in the case of measuring CEA concentration in pure pancreatic juice collected directly from a catheter inserted into the pancreatic duct after stimulating secretion of pancreatic juice.

Aspects of the present invention are indicated below.
(1) A method for detecting a marker for pancreatic disease, comprising: detecting a marker for pancreatic disease in duodenal juice that is collected from a subject and contains spontaneously discharged pancreatic juice.
(2) The method for detecting a marker for pancreatic disease described in (1) above, wherein the detecting of the marker for pancreatic disease comprises measuring the marker concentration and judging whether or not the concentration is equal to or greater than a prescribed threshold value.
(3) The method for detecting a pancreatic cancer marker described in (1) or (2) above, wherein the marker for pancreatic disease is CEA.
(4) The method for detecting a marker for pancreatic disease described in (2) above, wherein the marker for pancreatic disease is CEA,
   the pancreatic disease is pancreatic cancer, and
   the threshold value is a concentration within the range of 50 ng/mL to 200 ng/mL.
(5) The method for detecting a marker for pancreatic disease described in any of (1) to (4) above, wherein the duodenal juice is duodenal juice that has been stored in a state a protease inhibitor being added thereto.
(6) The method for detecting a marker for pancreatic disease described in any of (1) to (5) above, wherein the measurement method is immunochromatography or ELISA.
(7) A kit for measuring a marker for pancreatic disease in duodenal juice containing spontaneously discharged pancreatic juice, comprising a film on which is immobilized a first antibody to the marker for pancreatic disease.
(8) The kit for measuring a marker for pancreatic disease described in (7) above, further comprising a second antibody used to label the marker for pancreatic disease.
(9) The kit for measuring a marker for pancreatic disease described in (7) or (8) above, wherein the film is a test strip for immunochromatography.
(10) The kit for measuring a marker for pancreatic disease described in any of (7) to (9) above, further comprising a container for storing duodenal juice preliminarily containing a protease inhibitor.
(11) The kit for measuring a marker for pancreatic disease described in any of (7) to (10), wherein the marker for pancreatic disease is CEA.

### Effects of the Invention

The method for detecting a marker for pancreatic disease of one aspect of the present invention enables a marker for pancreatic disease such as CEA to be detected at a high level of accuracy comparable to the case of measuring CEA concentration in pure pancreatic juice collected directly from the pancreatic duct after administering a pancreatic juice secretion stimulator, without using a pancreatic juice secretion stimulator and without requiring the insertion of a catheter into the pancreatic duct. In other words, in addition to the absence of the risk of complications accompanying the procedure for inserting a catheter into the pancreatic duct, since the method for detecting a marker for pancreatic disease of the present invention can be carried out more easily and at lower cost in comparison with conventional methods, it enables pancreatic juice examinations to be performed more safely even on patients in which pancreatic disease is not suspected. Thus, the method for detecting a marker for pancreatic disease of the present invention can be expected to enable testing for pancreatic cancer to be applied over a wide range in routine medical examinations.

### BRIEF DESCRIPTION OF THE DRAWINGS

FIG. 1 is a graph showing the results of measuring p-amylase concentrations in spontaneously discharged duodenal juice (before secretin) and duodenal juice discharged after stimulation with secretin (after secretin) in Example 1.
FIG. 2 is a graph showing CEA concentrations in spontaneously discharged duodenal juice for various diseases in Example 1.
FIG. 3 is a graph showing CEA concentrations in duodenal juice discharged after stimulation with secretin for various diseases in Example 1.
FIG. 4 is a graph showing CEA concentrations in spontaneously discharged (absence of administration of secretin) for various diseases in Example 1.
FIG. 5 is a graph showing the results of analyzing receiver operating characteristics (ROC) in the case of using concentrations of CEA in spontaneously discharged duodenal juice as a marker for identifying pancreatic cancer and benign tumors (SCN) in Example 1.

### BEST MODE FOR CARRYING OUT THE INVENTION

In the present invention and description of the present application, a marker for pancreatic disease refers to various types of biomolecules such as proteins, nucleic acids, lipids or cells contained in pancreatic juice for which concentration in pancreatic juice increases significantly in patients afflicted with a pancreatic disease as compared with patients not afflicted with a pancreatic disease. Furthermore, persons not afflicted with a pancreatic disease are not limited to healthy individuals, but rather include patients afflicted with diseases other than a pancreatic disease. In addition, examples of pancreatic diseases include pancreatic cancer, intraductal papillary mucinous neoplasm (IPMN), mucinous cystic neoplasm (MCN), serous cystic neoplasm (SCN), neuroendocrine tumor (NET), chronic pancreatitis (CP) and acute pancreatitis.

Pancreatic juice is a bodily fluid discharged from the pancreatic duct into intestinal tract at the duodenum. Duodenal juice (intestinal juice present in the intestinal tract at the duodenum) contains, in addition to pancreatic juice, bile similarly discharged from the papilla, duodenal secretions discharged from the duodenal wall, and blood. In addition, in the present invention and description of the present application, spontaneously discharged pancreatic juice refers to pancreatic juice that has been discharged from the pancreatic duct into the intestinal tract at the duodenum without administering a pancreatic juice secretion stimulator such as secretin.

Since pancreatic juice is a digestive juice, it contains various digestive enzymes. Although these digestive enzymes are present in an inactive state in the pancreas, they are known to be activated after having been discharged into the duodenum. As a result of activation of these various digestive enzymes, various biomolecules such as proteins, nucleic acids, lipids and cells contained in pancreatic juice end up being decomposed and denatured following discharge into the duodenum. Consequently, when testing for a target biomolecule in pancreatic juice, it was not considered to be possible to obtain accurate measurements in the case of using pancreatic juice discharged into the duodenum as a measurement sample.

Consequently, it was conventionally thought to be necessary to sample pure pancreatic juice prior to being discharged into the intestinal tract at the duodenum directly from the pancreatic duct by inserting a catheter into the papillary region of the pancreas in order to test for pancreatic disease markers present in pancreatic juice at a level of accuracy required for clinical laboratory testing (see, for example, Non-Patent Documents 3 and 4). In addition, in the case of using duodenal juice for the measurement sample, it was also thought to be necessary to administer a pancreatic juice secretion stimulator such as secretin to cause an adequate amount of pancreatic juice to be discharged into the duodenum as a result of this stimulation (see, for example, Non-Patent Documents 1 and 2).

Despite these findings of the prior art, it was clearly determined by the inventors of the present invention that the concentration of a marker for pancreatic disease in duodenal juice containing spontaneously discharged pancreatic juice reflects the presence or absence of affliction with a pancreatic disease as well as the magnitude of the risk of the occurrence of pancreatic disease in the same manner the concentration of a marker for pancreatic disease in pure pancreatic juice collected directly from the pancreatic duct following stimulation with a pancreatic juice secretion stimulator. In other words, the concentration of a marker for pancreatic disease in duodenal juice containing spontaneously discharged pancreatic juice is higher in persons afflicted with a pancreatic disease in comparison with persons not afflicted with a pancreatic disease. Consequently, by detecting a marker for pancreatic disease in duodenal juice collected from a subject and then measuring the concentration thereof, whether or not the subject is afflicted with a pancreatic disease and the risk of the occurrence of a pancreatic disease can be investigated at a high level of accuracy comparable to the most accurate and reliable methods of the prior art (consisting of using as a measurement sample pure pancreatic juice collected directly from the pancreatic duct following stimulation with a pancreatic juice secretion stimulator).

Namely, the method for detecting a marker for pancreatic disease of an aspect of the present invention (to be referred to as the method for detecting a marker for pancreatic disease of the present invention) is characterized by measuring the concentration of a marker for pancreatic disease in duodenal juice collected from a subject that contains spontaneously discharged pancreatic juice. In the method for detecting a marker for pancreatic disease of the present invention, the method can be carried out at lower cost since stimulation with a pancreatic juice secretion stimulator is not required. In addition, although pancreatic juice is typically collected after 30 to 45 minutes have elapsed following stimulation in the case of collecting pancreatic juice following stimulation with a pancreatic juice secretion stimulator; in the present invention, since duodenal juice is collected that has accumulated in the intestinal tract at the duodenum, a sample can be collected in a shorter period of time (for example, on the order of several minutes to several tens of minutes). Moreover, since a catheter is not inserted into the pancreatic duct of the papillary region, duodenal juice used as measurement sample for detecting a marker for pancreatic disease can be collected less invasively and more easily. Namely, since the method for detecting a marker for pancreatic disease of the present invention is free of the risk of complications, testing for pancreatic disease markers, which was previously only able to be performed on patients clearly determined to have a pancreatic disease in consideration of the risk thereof, can be performed more safely even on subjects for which the presence or absence of the onset of a pancreatic disease is unknown.

The duodenal juice used in the method for detecting a marker for pancreatic disease of the present invention is juice that has been collected from the intestinal tract at the duodenum of a subject who has not been administered a pancreatic juice secretion stimulator. Although the duodenal juice may be duodenal juice collected from any location in the intestinal tract of the duodenum, it is preferably duodenal juice present at the second portion or third portion of the duodenum. This is because the first portion of the duodenum is directly connected to the pyloric region of the stomach, thereby resulting in the possibility of contamination by gastric juice, while also potentially making collection difficult since it is comparatively difficult to immobilize the endoscope for the purpose of collection.

There are no particular limitations on the method used to collect duodenal juice provided it enables collection of juice that has accumulated in the intestinal tract of the duodenum. For example, duodenal juice can be collected by an aspiration means such as a syringe or vacuum pump connected to an endoscopic catheter. More specifically, an endoscope is inserted through the oral cavity into the duodenum, and duodenal juice present at the second portion and third portion of the duodenum is aspirated and collected using a catheter that has been inserted by passing through the forceps channel of the endoscope. For example, after performing an endoscopic examination of the stomach and duodenum (upper gastrointestinal endoscopy) in the form of a so-called gastroscopy, duodenal juice accumulated in the intestinal tract at the duodenum may be collected, and testing for pancreatic disease markers present in the resulting duodenal juice may be performed.

Although testing on collected duodenal juice for measuring a marker for pancreatic disease is preferably performed immediately or within several hours after collection, the duodenal juice may be subjected to testing for measuring the concentration of a marker for pancreatic disease after having been stored for a prescribed amount of time after collection. The collected duodenal juice may be stored refrigerated or frozen, may be stored at room temperature, or may be stored as a frozen powder by subjecting to freeze-drying treatment. In addition, the duodenal juice can be stored after mixing with a substance that inhibits degradation by digestive enzymes present in the duodenal juice. More specifically, the duodenal juice is preferably mixed with a protease inhibitor against proteases that decompose pancreatic disease marker proteins, and is particularly preferably mixed with an inhibitor for a serine protease present in large amounts in pancreatic juice. Protease inhibitors and the like may be mixed after having collected duodenal juice from the body, or duodenal juice can be collected in a container preliminarily containing a protease inhibitor and the like.

The collected duodenal juice may be used directly in testing for measuring a marker for pancreatic disease, or may be used after having separated and removed cells and other solid fractions by centrifugation and the like. In addition, diluted duodenal juice obtained by diluting collected duodenal juice with a suitable buffer may be used, or duodenal juice obtained by adding various types of additives to duodenal juice or a dilution thereof may be used in testing for measuring a marker for pancreatic disease. Examples of additives include surfactants, protease inhibitors, nuclease inhibitors, pH adjusters and pH indicators.

There are no particular limitations on the marker for pancreatic disease detected in the method for detecting a marker for pancreatic disease of the present invention provided it is a biomolecule for which the concentration thereof in pancreatic juice increases significantly in patients afflicted with a pancreatic disease in comparison with patients not afflicted with a pancreatic disease. In the present invention, the marker for pancreatic disease is preferably a biomolecule such as a glycoprotein or enzyme that is resistant to the effects of digestive enzymes contained in duodenal juice. Examples of pancreatic disease markers detected in the method for detecting a marker for pancreatic disease of the present invention include glycoproteins in the form of CEA, CA19-9 (see, for example, Non-Patent Document 3) or MUC-1 (KL-6) (see, for example, Non-Patent Document 3), and enzymes in the form of matrix metalloproteinase-2 (MMP-2) (see, for example, Pancreas, 2002, Vol. 24, No. 54, pp. 344-347) or matrix metalloproteinase-7 (MMP-7). Other examples include substances such as S100P for which the concentration thereof increases significantly in the pancreas in patients afflicted with a pancreatic disease.

The marker for pancreatic disease detected in the method for detecting a marker for pancreatic disease of the present invention is preferably CEA. The presence or absence of occurrence, or the risk of occurrence, of diseases such as pancreatic cancer, IPMN, MCN, chronic pancreatitis or acute pancreatitis can be investigated by using CEA concentration in duodenal juice containing spontaneously discharged pancreatic juice as an indicator thereof.

In the method for detecting a marker for pancreatic disease of the present invention, a marker for pancreatic disease is detected in duodenal juice using an antigen-antibody reaction. In the case of detecting by using an antigen-antibody reaction, an antibody immobilized on a solid phase, namely an antibody to a marker for pancreatic disease (or antigen in the case the marker for pancreatic disease is an antibody), is contacted with duodenal juice, and the antigen-antibody complex that has formed is detected on that solid phase. For example, an antibody to a marker for pancreatic disease (or antigen in the case the marker for pancreatic disease is an antibody) is preliminarily immobilized on a solid phase, duodenal juice is contacted with this solid phase, the immobilized antibody (or antigen) is allowed to bind to the marker for pancreatic disease, and the complex that has formed is detected. Conversely, a biomolecule in duodenal juice is immobilized on a solid phase, an antibody to a marker for pancreatic disease (or antigen in the case the marker for pancreatic disease is an antibody) is contacted with this solid phase and allowed to bind with the immobilized pancreatic disease marker, and the complex that has formed is detected.

Examples of the solid phase used to immobilize antigen or antibody include polymer membranes such as a nitrocellulose membrane, gels such as agarose gel or SDS-PAGE gel, inner surfaces of containers such as plastic tubes or microplates, and beads such as latex beads or magnetic beads. Examples of methods used to detect the antigen-antibody complex formed with respect to antigen or antibody immobilized on the solid phase include immunochromatography, western blotting, dot plotting, slot blotting, immunoelectrophoresis, immunodiffusion, ELISA, immunoagglutination methods using latex beads, immunoassay methods using magnetic beads, and SPR. Methods used to immobilize antigen or antibody as well as these methods can be carried out according to ordinary methods with the exception of using duodenal juice as the measurement target and using antibody to a marker for pancreatic disease (or antigen in the case the marker for pancreatic disease is an antibody).

The antigen-antibody reaction can also be detected in a liquid phase. In the case of detecting in a liquid phase, binding between the labeled antibody and antigen can be measured by FCS or FRET and the like.

In addition, the presence of antigen can also be measured by measuring molecular weight by TOF-MS and the like.

In embodiments of the present invention, the marker for pancreatic disease is preferably detected by immunochromatography or ELISA, and more preferably detected by immunochromatography, in consideration of speed, simplicity and ease of quantitative detection. For example, duodenal juice is dropped onto a nitrocellulose membrane having an antibody to a marker for pancreatic disease preliminarily immobilized on the surface thereof, the marker for pancreatic disease in this duodenal juice binds with the immobilized antibody, and the complex that has formed is then detected. By using a labeled antibody pre-labeled with a fluorescent substance, colloidal gold or enzyme and the like as an antibody that binds with this complex, this complex can be detected in the form of a band.

The concentration of pancreatic disease marker in duodenal juice can be measured qualitatively by visually comparing the intensity of the band detected using duodenal juice with a calibration color chart prepared using known concentrations of pancreatic disease markers. In addition, concentration of the marker for pancreatic disease can also be easily determined by optically measuring band intensity and similarly comparing with the intensities of bands detected using known concentrations of pancreatic disease markers. Moreover, a calibration curve representing the relationship between band intensity and pancreatic disease marker concentration can be prepared in advance, the intensity of a band detected using duodenal juice is measured optically, and the concentration of the marker for pancreatic disease in the duodenal juice can be determined by converting from the calibration curve.

The concentration of a marker for pancreatic disease in duodenal juice containing spontaneously discharged pancreatic juice tends to be higher in persons afflicted with a pancreatic disease than in persons not afflicted with a pancreatic disease. Consequently, the possibility of a subject being afflicted with a pancreatic disease can be assessed by using the concentration of that pancreatic disease marker in the duodenal juice of that subject. A threshold value is preliminarily set that distinguishes between persons afflicted with a pancreatic disease and persons not afflicted with a pancreatic disease, the concentration of a marker for pancreatic disease in the duodenal juice of a subject is measured, and a judgment is made as to whether or not that concentration is equal to or greater than the prescribed threshold value. In the case the concentration is equal to or greater than the prescribed threshold value, the subject is judged to have a high possibility of being afflicted with that pancreatic disease. In this manner, pancreatic disease can be detected by measuring the concentration of a marker for pancreatic disease in duodenal juice containing spontaneously discharged pancreatic juice, and comparing that concentration with a prescribed threshold value.

The threshold value used to distinguish between persons afflicted and not afflicted with a pancreatic disease can be set experimentally in consideration of such factors as the type of pancreatic disease marker or the method used to detect the marker for pancreatic disease. For example, the detection method of the present invention is carried out on duodenal juice collected from group known not to be afflicted with a pancreatic disease and on duodenal juice collected from a group known to be afflicted with this disease, the concentrations of the marker for pancreatic disease in spontaneously discharged duodenal juice are measured, and the threshold value is suitably set by comparing the measured values of both groups.

For example, in the case of using CEA as a marker for pancreatic disease, the threshold value that distinguishes between persons afflicted and not afflicted with pancreatic cancer (persons afflicted with a benign tumor in the form of SCN) is preferably set to a concentration within the range of 50 ng/mL to 200 ng/mL, and more preferably to a concentration within the range of 100 ng/mL to 150 ng/mL. By setting the threshold value within these ranges, whether or not a subject is afflicted with pancreatic cancer can be identified with clinically adequate sensitivity and specificity by using the method for detecting a marker for pancreatic disease of the present invention. In particular, in addition to being able to reliably detect pancreatic cancer, diseases such as IPMN, which is associated with a high risk for pancreatic cancer, or MCN, which is treatable, can also be detected, enabling a wide range of asymptomatic pancreatic diseases to be detected. In addition, since there is a high possibility that pancreatic cancer is in an advanced state if CEA concentration is high, such cases can be judged to promptly require follow-up examinations and commencement of treatment.

More specifically, in the case the marker for pancreatic disease is CEA, duodenal juice is dropped onto a nitrocellulose membrane (for immunochromatography), obtained by preliminarily immobilizing anti-CEA antibody on the surface of the membrane, along with a labeled anti-CEA antibody having a different epitope sites than the antibody immobilized on the membrane, and a complex composed of the immobilized anti-CEA antibody, CEA and the labeled CEA antibody is formed on the surface of the nitrocellulose membrane. The band that has formed is detected as a band with the label in the labeled CEA antibody. In immunochromatography, CEA concentration can also be quantified based on band intensity by visually comparing with band intensities of a color chart depicting bands obtained in the case of having added prescribed concentrations of CEA. In addition, CEA concentration can also be determined by optically measuring band intensity and converting from a prescribed calibration curve. In immunochromatography, for example, band intensity is compared with a color chart depicting bands corresponding to CEA concentrations of 50 ng/mL and 200 mg/mL, and in the case the band intensity is intermediate to these two bands, or in the case band intensity is greater than the 200 mg/mL band, the result is judged to be positive (indicating a high possibility of the subject from which the duodenal juice used for measurement was collected being afflicted with a pancreatic disease such as pancreatic cancer, IPMN, MCN, chronic pancreatitis or acute pancreatitis, and particularly pancreatic cancer).

In addition, in diseases such as IPMN or MCN that are frequently benign, CEA concentrations are presumed to be comparatively low, while CEA concentrations in advanced pancreatic cancer and advanced chronic pancreatitis are presumed to be high. Consequently, the magnitude of the possibility of being afflicted with a specific pancreatic disease other than pancreatic cancer can be expected to be assessed by setting a suitable threshold value.

In this manner, an assessment of a positive or negative result (assessment as to whether a subject is afflicted with a pancreatic disease) can be made both rapidly and easily in the endoscopy room where duodenal juice is collected by measuring CEA concentration in spontaneously discharged duodenal juice using a rapid-test kit in the manner of an immunochromatography kit. Thus, since use of the method for detecting a marker for pancreatic disease of the present invention enables screening tests for pancreatic cancer to be performed safely, with high performance, and rapidly, the possibility of having pancreatic cancer, which advances rapidly and can be fatal in several months, can be rapidly evaluated following collection of duodenal juice, thereby making it possible to promptly begin pancreatic cancer treatment by providing motivation and enabling appointments for follow-up testing to be made quickly.

The method for detecting a marker for pancreatic disease of the present invention can be carried out even more easily by incorporating reagents, apparatuses and so forth used in a method for detecting a marker for pancreatic disease in duodenal juice in a kit. In another aspect thereof, a kit for measuring a marker for pancreatic disease in duodenal juice containing spontaneously discharged pancreatic juice (to be referred to as the kit for measuring a marker for pancreatic disease of the present invention) preferably contains a solid phase having an antibody to a marker for pancreatic disease (or antigen in the case the marker for pancreatic disease is an antibody) immobilized thereon, an example of which is a membrane having a first antibody to a marker for pancreatic disease immobilized thereon. An example of this membrane is a test strip used for immunochromatography. This measurement kit preferably also has a second antibody used to label the marker for pancreatic disease. In addition, this measurement kit preferably further comprises a container for storing duodenal juice preliminarily containing a protease inhibitor.

### Examples

Although the following provides a more detailed explanation of the present invention by indicating examples thereof, the present invention is not limited to the following examples.

### [Example 1]

Duodenal juice was collected from 73 cases consisting of patients suspected of having a pancreatic disease followed by measurement of CEA concentration.

After having the patients fast overnight, an endoscope was inserted to the second portion of the duodenum and duodenal juice accumulated near the surface of the duodenal wall throughout the second portion and third portion was collected using a pancreatography catheter (Olympus, product name: PR-130Q). Duodenal juice was collected by aspirating with a syringe connected to the operative side of the catheter outside the endoscope. An average of about 1 mL of duodenal juice was able to be collected in about 2 to 3 minutes. The endoscope passed through to the gastric side in the first portion of the duodenum, preventing stable collection.

After collecting spontaneously discharged duodenal juice, secretin (ChiRhoClin) was intravenously injected into 47 of the 73 cases, and duodenal juice accumulated near the surface of the duodenal wall throughout the second portion and third portion was similarly collected 2 to 3 minutes later. Approximately 1.5 mL to 2 mL of duodenal juice was able to be collected roughly 2 to 3 minutes after stimulating with secretin.

Subsequently, each of the patients underwent diagnostic imaging and pathological examinations and a final diagnosis was made. As a result of final diagnosis, there were 41 cases of pancreatic cancer, 17 cases of IPMN, 2 cases of MCN, 6 cases of SCN, 3 cases of NET and 4 cases of chronic pancreatitis (CP) among the 73 cases. A breakdown of the 47 of 73 cases from which both spontaneously discharged duodenal juice and duodenal juice following stimulation with secretin were able to be collected consisted of 29 cases of pancreatic cancer, 7 cases of IPMN, 2 cases of MCN, 3 cases of SCN, 1 case of NET and 1 case of chronic pancreatitis (CP).

The p-amylase and CEA concentrations were each measured in the two types of duodenal juice (spontaneously discharged duodenal juice and duodenal juice discharged after secretin stimulation) collected from the 47 cases from which both spontaneously discharged duodenal juice and duodenal juice following stimulation with secretin were able to be collected. The p-amylase concentrations were measured by an enzymatic method (Gal-G2-CNP substrate method). In addition, CEA concentrations were measured by ELISA using a commercially available kit (IBL).

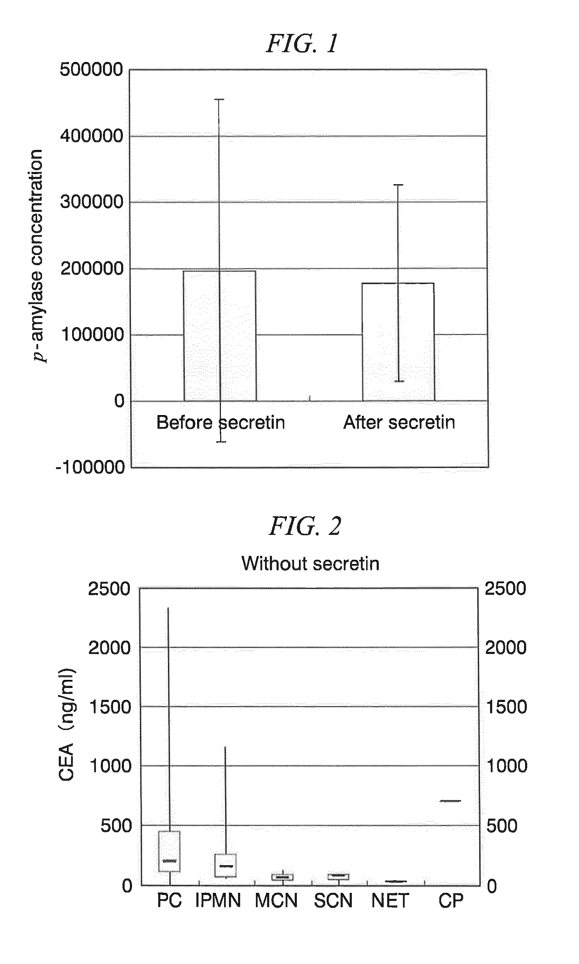

FIG. 1 shows the results of measuring p-amylase concentration in each duodenal juice sample collected from the 47 cases from which both spontaneously discharged duodenal juice (before secretin stimulation) and duodenal juice following stimulation with secretin (after secretin) were able to be collected. The p-amylase was determined to be contained in the duodenal juice before administration of secretin to about the same degree as the duodenal juice following administration of secretin. Since p-amylase originates in pancreatic juice, pancreas-derived pancreatic juice was confirmed to be spontaneously discharged and contained in the duodenal juice, and components derived from pancreatic juice were confirmed to be able to be detected from spontaneously discharged duodenal juice as well.

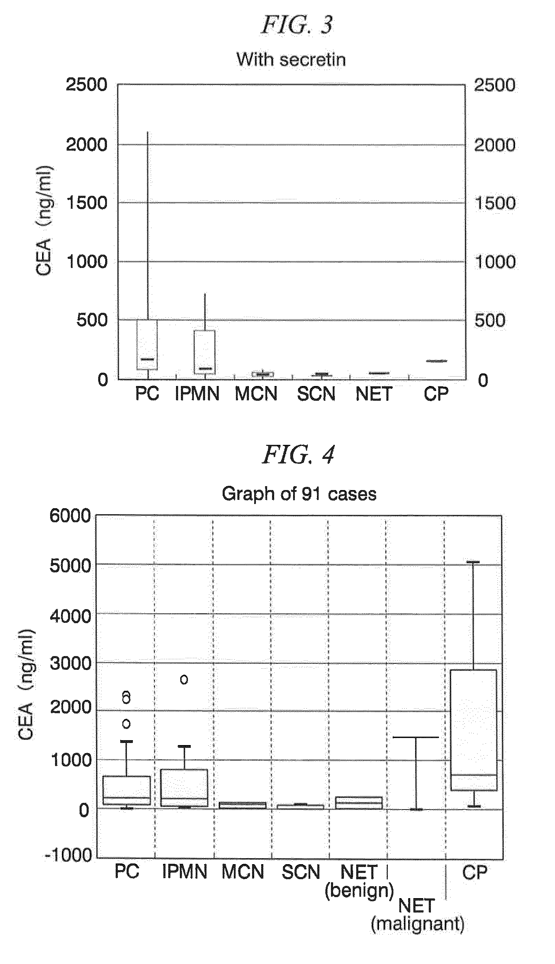

FIG. 2, FIG. 3 and Table 1 indicate CEA concentrations in the duodenal juice of the 47 cases from which both spontaneously discharged duodenal juice and duodenal juice following stimulation with secretin were able to be collected for various diseases. FIG. 2 indicates CEA concentrations in spontaneously discharged duodenal juice, while FIG. 3 indicates CEA concentrations in duodenal juice discharged following stimulation with secretin. As a result, the CEA concentrations in duodenal juice for various diseases were roughly equal for spontaneously discharged (absence of administration of secretin) duodenal juice and duodenal juice discharged following administration of secretin. In addition, even when compared with previous reports such as that of Non-Patent

Document 4, the CEA concentrations in spontaneously discharged (absence of administration of secretin) duodenal juice in the present example were in a similar concentration range as the CEA concentrations in duodenal juice collected by cannulation of the papillary region. On the basis of these results, even in the case of measuring CEA concentration in spontaneously discharged duodenal juice, effects enabling differentiation of pancreatic diseases were determined to be obtained that were similar to effects in the case of measuring CEA concentration in pure pancreatic juice.

**[Table 1]**

| | CEA Concentration in Duodenal Juice (ng/ml) | | | | | | |
|---|---|---|---|---|---|---|---|
| | | PC | IPMN | MCN | SCN | NET | CP |
| Without secretin | Mean | 457.0 | 289.3 | 60.5 | 61.5 | 33.8 | 701.4 |
| | SD | 578.50 | 392.24 | 84.14 | 52.50 | -- | -- |
| With secretin | Mean | 350.9 | 242.9 | 40.2 | 30.6 | 47.8 | 156.3 |
| | SD | 454.82 | 313.60 | 55.42 | 22.07 | -- | -- |
| N | | 29 | 6 | 2 | 3 | 1 | 1 |

The CEA concentrations in spontaneously discharged (absence of administration of secretin) duodenal juice of all 73 cases are shown in FIG. 4 and Table 2.

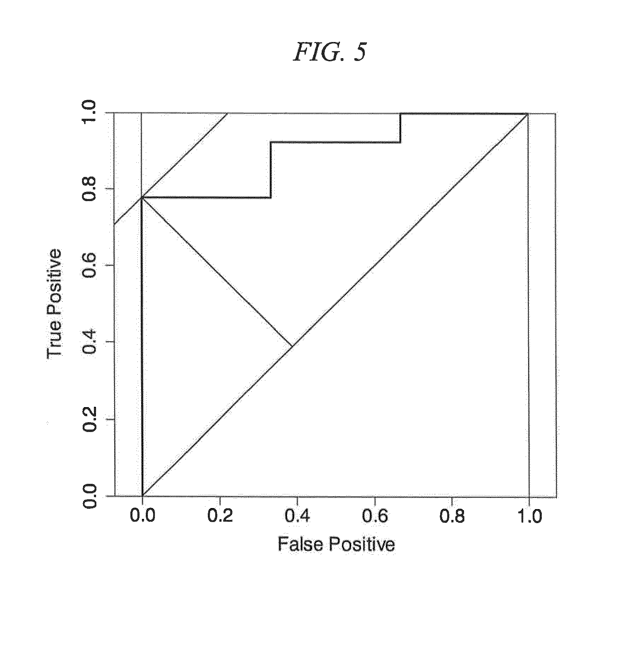

In addition, CEA concentrations in the duodenal juice demonstrated high values in cases of pancreatic cancer and demonstrated high values in cases of the treatable pancreatic diseases of IPMN and chronic pancreatitis (CP) in nearly the same manner as the results shown in FIG. 2. CEA concentrations were also higher in malignant NET data than in benign NET data. Values were low in cases of SCN benign tumors, for which progress is required to be monitored, which also coincided with the results of FIG. 2. Therefore, a receiver operating characteristics (ROC) analysis was performed in order to determine whether or not CEA concentration in spontaneously discharged duodenal juice can be used as a marker for differentiating pancreatic cancer from benign tumors (SCN in the present invention). The ROC curve was prepared using PASW Statistics Ver. 18 (IBM). The analysis results are shown in FIG. 5. According to these results, the cutoff value (threshold value) for differentiating between pancreatic cancer and benign tumors was nearly equal to the cases of CEA concentration in pure pancreatic juice and CEA concentration in duodenal juice collected after administering secretin. The detection sensitivity of pancreatic cancer in the present example was 71% and the detection specificity was 100%, thereby demonstrating performance that is superior to the use of CEA concentration in pure pancreatic juice as an indicator as previously reported.

**[Table 2]**

| | CEA Concentrations in Spontaneously Discharged Duodenal Juice (Absence of Secretin Stimulation) | | | | | | |
|---|---|---|---|---|---|---|---|
| | PC | IPMN | MCN | SCN | NET (benign) | NET (malignant) | CP |
| Mean | 526.6 | 527.9 | 60.5 | 59.8 | 140.7 | 1499.7 | 1629.2 |
| SD | 647.36 | 676.48 | 84.14 | 52.21 | 151.12 | 8.5 | 2292.30 |
| N | 41 | 17 | 2 | 6 | 2 | 1 | 4 |

Next, sensitivity and specificity were calculated for respective cutoff values by setting the cutoff value for differentiating between pancreatic cancer and benign tumor (SCN in the present invention) within the range of 30 ng/mL to 250 ng/mL. The calculation results are shown in Table 3. As a result, in the case of setting the cutoff value from 100 ng/mL to 150 ng/mL, sensitivity was 70% or higher even if specificity is 80% or higher, thereby obtaining a level of sensitivity useful for use in testing. In addition, in the case of setting to this cutoff value, pancreatic cancer can be detected from among diseases such as MCN, thereby making it possible to anticipate testing at an even higher level of specificity for pancreatic cancer. In addition, NET can also be expected to be able to be assessed in addition to pancreatic cancer. On the other hand, if the cutoff value is set to 50 ng/mL, specificity is 50%, and although specificity is roughly only half that in the case of setting to 100 ng/mL to 150 ng/mL, sensitivity becomes 93%, thereby making it possible to conduct testing for pancreatic cancer with extremely high sensitivity.

**[Table 3]**

| Cutoff value (ng/mL) | Sensitivity (%) | Specificity (%) |
|---|---|---|
| 250 | 44 | 100 |
| 230 | 49 | 100 |
| 200 | 54 | 100 |
| 180 | 63 | 100 |
| 150 | 71 | 100 |
| 120 | 71 | 83 |
| 100 | 76 | 83 |
| 70 | 85 | 50 |
| 50 | 93 | 50 |
| 30 | 93 | 33 |

### [Example 2]

CEA was detected in the naturally discharged duodenal juice specimens used in Example 1 using a commercially available CEA immunochromatography kit (trade name: Rana Manmo Card, Nippon Kayaku). Visual assessments of CEA concentration were made by comparing the intensity of the immunochromatography band detected for each duodenal juice specimen with a band color chart provided with the kit.

As a result, a correlation was observed between band intensity of the duodenal juice specimens and measured values for CEA concentration determined by ELISA in the case of comparing with the 100 ng/mL and 400 ng/mL color samples, thereby confirming that CEA concentration in duodenal juice can be also be measured by immunochromatography.

### [Example 3]

Protease inhibitor (trade name: Complete, Roche) was preliminarily filled into a syringe connected to the operative side of a catheter outside an endoscope, and duodenal juice was aspirated through the catheter from 47 of the patients of Example 1. The aspirated duodenal juice was mixed with the protease inhibitor inside the syringe followed by transferring to a tube and placing in frozen storage. Duodenal juice aspirated through a catheter into a syringe not containing protease inhibitor in advance was similarly transferred to a tube and placed in frozen storage for use as a control.

CEA concentration and S100P concentration were measured for each duodenal juice specimen following storage. CEA concentrations were measured in the same manner as Example 1. In addition, S100P concentrations were measured by ELISA using a commercially available kit (Cyclex).

**[Table 4]**

| | CEA concentration in duodenal juice (ng/ml) | | | | | | |
|---|---|---|---|---|---|---|---|
| | | PC | IPMN | MCN | SCN | NET | CP |
| Without inhibitor | Mean | 457.0 | 289.3 | 60.5 | 61.5 | 33.8 | 701.4 |
| | SD | 578.50 | 392.24 | 84.14 | 52.50 | -- | -- |
| With inhibitor | Mean | 443.4 | 458.8 | -- | 66.7 | 49.3 | 246.6 |
| | SD | 530.88 | 525.45 | -- | 50.34 | -- | -- |

**[Table 5**

| | S100P concentration in duodenal juice (ng/ml) | | | | | | |
|---|---|---|---|---|---|---|---|
| | | PC | IPMN | MCN | SCN | NET | CP |
| Without inhibitor | Mean | 22991.5 | 8124.2 | 452.2 | 0.0 | 0.0 | 9476.9 |
| | SD | 40702.84 | 18688.69 | 666.26 | -- | -- | -- |
| With inhibitor | Mean | 39289.7 | 20554.1 | 11660.0 | 96.8 | 8133.3 | 18887.2 |
| | SD | 62998.73 | 36631.07 | -- | -- | -- | -- |

The results of measuring CEA concentration and S100P concentration in duodenal juice are shown for various diseases in Tables 4 and 5. As a result, CEA concentrations were determined to not demonstrate significantly large differences between those in duodenal juice stored following the addition of protease inhibitor ("With inhibitor" in Table 4) and duodenal juice stored without adding protease inhibitor ("Without inhibitor" in Table 4). On the other hand, S100P concentrations were significantly higher in duodenal juice stored following the addition of protease inhibitor ("With inhibitor" in Table 5) than duodenal juice stored without adding protease inhibitor ("Without inhibitor" in Table 5). When confirming the results for individual cases, there were several specimens that were unable to be measured in the case of not adding protease inhibitor that were able to be measured following addition of protease inhibitor. On the basis of these results, preliminarily mixing a protease inhibitor with duodenal juice collected from the body was confirmed to improve detection accuracy of S100P since digestion of S100P in duodenal juice was suppressed.

### INDUSTRIAL APPLICABILITY

In addition to being safe in terms of eliminating risks such as symptoms associated with acute pancreatitis, since the method for detecting a marker for pancreatic disease of the present invention makes it possible to detect a marker for pancreatic disease with a level of accuracy comparable to the case of measuring CEA concentration in pure pancreatic juice collected directly from a catheter inserted into the pancreatic duct after stimulating secretion of pancreatic juice, the method of the present invention can be used in fields relating to the analysis of pancreatic juice, and particularly in the field of clinical laboratory testing and the like.

## Claims

1. A method for detecting a marker for pancreatic disease, comprising:
detecting a marker for pancreatic disease in duodenal juice that is collected from a subject and contains spontaneously discharged pancreatic juice.

2. The method for detecting a marker for pancreatic disease according to claim 1, wherein the detecting of the marker for pancreatic disease comprises measuring the marker concentration and judging whether or not the concentration is equal to or greater than a prescribed threshold value.

3. The method for detecting a marker for pancreatic disease according to claim 1 or 2, wherein the marker for pancreatic disease is CEA.

4. The method for detecting a marker for pancreatic disease according to claim 2, wherein the marker for pancreatic disease is CEA,
the pancreatic disease is pancreatic cancer, and
the threshold value is a concentration within the range of 50 ng/mL to 200 ng/mL.

5. The method for detecting a marker for pancreatic disease according to any of claims 1 to 4, wherein the duodenal juice is duodenal juice that has been stored in a state protease inhibitor being added thereto.

6. The method for detecting a marker for pancreatic disease according to any one of claims 1 to 5, wherein the measurement method is immunochromatography or ELISA.

7. A kit for measuring a marker for pancreatic disease in duodenal juice containing spontaneously discharged pancreatic juice, comprising a film on which is immobilized a first antibody to the marker for pancreatic disease.

8. The kit for measuring a marker for pancreatic disease according to claim 7, further comprising a second antibody used to label the marker for pancreatic disease.

9. The kit for measuring a marker for pancreatic disease according to claim 7 or 8, wherein the film is a test strip for immunochromatography.

10. The kit for measuring a marker for pancreatic disease according to any of claims 7 to 9, further comprising a container for storing duodenal juice preliminarily containing a protease inhibitor.

11. The kit for measuring a marker for pancreatic disease according to any of claims 7 to 10, wherein the marker for pancreatic disease is CEA.
